# EUROPÄISCHE PATENTSCHRIFT

(11) **EP 2 725 899 B1**
(45) Veröffentlichungstag und Bekanntmachung des Hinweises auf die Patenterteilung: **28.09.2016**
(21) Anmeldenummer: 12761703.3
(22) Anmeldetag: 28.06.2012
(51) Int. Cl.: A01N 25/22, A01N 47/02, A01N 53/00, A01P 7/02

(54) **MITTEL ZUR BEKÄMPFUNG VON PARASITEN AN TIEREN**
AGENT FOR COMBATING PARASITES ON ANIMALS
PRODUIT DE LUTTE ANTIPARASITAIRE POUR LES ANIMAUX

(30) Priorität: 30.06.2011 EP 11005341
(43) Veröffentlichungstag der Anmeldung: 07.05.2014
(73) Patentinhaber: Hansen-AB GmbH, 51371 Leverkusen (DE)
(72) Erfinder: KALBE, Jochen, 42799 Leichlingen (DE); HANSEN, Olaf, 51371 Leverkusen-Hitdorf (DE)
(74) Vertreter: Gille Hrabal
(86) Internationale Anmeldenummer: PCT/EP2012/002712
(87) Internationale Veröffentlichungsnummer: WO 2013/000572

(56) Entgegenhaltungen:
- WO-A2-2008/030385
- WO-A2-2009/027506
- WO-A2-2010/031584
- US-A1- 2003 187 029
- US-A1- 2011 071 193
- "238 deltamethrin" In: "The Pesticide Manual - A World Compendium - 15th Edition", 1. Januar 2009 (2009-01-01), British Crop Protection Council, Alton, Hampshire, GU34 2QD, United Kingdom, XP055013060, ISBN: 978-1-90-139618-8 Seiten 313-315, das ganze Dokument
- S A ET AL: 'Synthetic pyrethroid insecticides: a dermatological evaluation' BRITISH JOURNAL OF INDUSTRIAL MEDICINE, [Online] 01 Januar 1985, XP055166987 Gefunden im Internet: <URL:http://www.ncbi.nlm.nih.gov/pmc/articl es/PMC1007493/pdf/brjindmed00182-0003.pdf>

## Beschreibung

Die Erfindung betrifft Mittel zur Bekämpfung von Parasiten an Tieren, enthaltend Fipronil als einen Wirkstoff aus der Gruppe der Phenylpyrazole und Vitamin E oder ein Derivat davon, wie insbesondere Vitamin E Acetat, sowie Deltamethrin als einen weiteren Wirkstoff aus der Gruppe der Pyrethroide sowie gegebenenfalls zusätzlich weitere Wirk- und/oder Hilfsstoffe. Insbesondere betrifft die Erfindung die Verwendung derartiger Mittel zur Bekämpfung von Ektoparasiten wie insbesondere Flöhen, Zecken und Sandmücken bei Haustieren wie insbesondere bei Hunden, Katzen und Frettchen.

### EINLEITUNG UND STAND DER TECHNIK

Aus dem Stand der Technik sind zahlreiche Mittel zur Bekämpfung von Parasiten wie insbesondere Ektoparasiten auf Basis von Wirkstoffen aus der Gruppe der Phenylpyrazole, gegebenenfalls auch in Kombination mit weiteren Wirkstoffen, zum Beispiel aus der Gruppe der Pyrethroide, oder mit Hilfsstoffen wie Spreit- und Lösungsmitteln bekannt. Insbesondere ist aus dem Stand der Technik bekannt, derartige Mittel zur Bekämpfung von Parasiten mit Hilfsstoffen aus der Gruppe der Antioxidantien zu versetzen, was insbesondere bei Verwendung lipophiler bzw. Fett-, Öl- oder Wachshaltiger Hilfsstoffe oder Wirkstoffformulierungen von Bedeutung ist. Ein übliches und bekanntes Antioxidationsmittel ist unter anderem Tocopherol oder Tocopherolnicotinat aus der Gruppe der Vitamin E Verbindungen, welches dann üblicherweise in Mengen < 1 Gew,-% bzw. bis maximal 10 Gew.-% eingesetzt wird.

Insbesondere ist aus dem Stand der Technik auch bereits bekannt, dass Phenylpyrazol-Derivate wie Fipronil sowie Pyrethroid-Derivate wie Deltamethrin oder Flumethrin wirksame Mittel zur Bekämpfung von Bekämpfung von Parasiten bei Tieren sind.

Fipronil ist ein Phenylpyrazol-Derivat (1-[2,6-Cl₂-4-CF₃-phenyl]-3-CN-4-[SO-CF₃]-5-NH₂-pyrazol) und entspricht der allgemeinen Formel

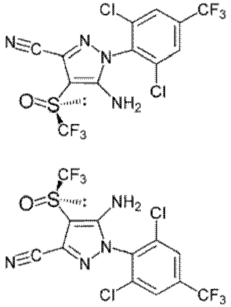

Fipronil findet insbesondere Anwendung in der Agrarwirtschaft und der Tiergesundheit bei der Bekämpfung von Parasiten wie Arthropoden, Nematoden, Helminthen und Protozoen aber auch gegen Ektoparasiten wie Flöhe, Läuse und Zecken und wird beispielsweise in den Patenten EP 2951 1 7, US 5,232,940, EP 352944 sowie der GB 2457734, genannt.

Flumethrin (α-Cyano(4-fluoro-3-phenoxyphenyl)methyl 3-[2-chloro-2-(4-chlorophenyl)ethenyl]-2,2-dimethylcyclopropanecarboxylate), entsprechend der allgemeinen Formel 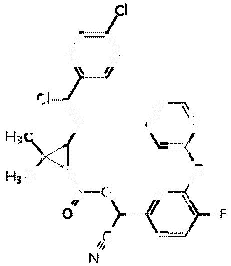 sowie Deltamethrin (auch Decamethrin oder (1R,3R)-[(S)-α-Cyano-3-phoxybenzyl-3-(2,2-dibromvinyl)]-2,2-dimethylcyclopropancarboxylat), entsprechend der allgemeinen Formel 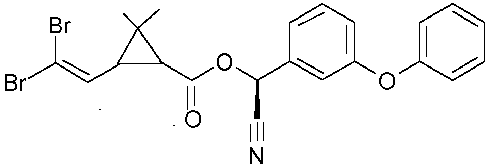 sind Pyrethroid-Derivate des Typs II und schnell wirkende Kontaktgifte (Nervengifte), die dazu führen, dass sich die Na⁺-Kanäle der Nervenzellen nicht mehr schließen, wodurch Na⁺-Ionen ungehindert in das Zellinnere hineinströmen und es zu unkontrollierbaren Nervenimpulsen kommt. Dadurch wird beispielsweise bei Arthropoden ein rasch einsetzender Knock-down-Effekt ausgelöst. Dieser Effekt ist im Vergleich zu den natürlichen Pyrethrinen aufgrund der besseren Stabilität von Flumethrin und Deltamethrin von wesentlich längerer Dauer. Sowohl Flumethrin als auch Deltamethrin besitzen außerdem ausgeprägte repellierende Eigenschaften. Diese beruhen auf einer Reizung taktiler Elemente in den Extremitäten (Fuss-Rückzieh-Effekt) der Arthropoden. Bei Zecken kommt es neben der abtötenden Wirkung auch zu einer Hemmung der Eiablage, sodass überlebende weibliche Zecken nicht in der Lage sind entwicklungsfähige Nachkommen zu produzieren.

Sowohl Flumethrin als auch Deltamethrin sind bekannt für ihre irritierende Wirkung auf empfindliche Hautpartien, Schleimhäute und Augen, wodurch es zu lokalisierten Pruritus und Erythembildungen einhergehend mit Alopeziebildung kommen kann.

Um eine Verbesserung von Schädlingsbekämpfungsmitteln sowohl hinsichtlich der akuten Wirksamkeit als auch der Wirkungsdauer zu erzielen sind in der Vergangenheit zahlreiche Kombinationspräparate entwickelt worden. So sind beispielsweise auch verschiedene Kombinationspräparate auf Basis von Pyrazolen wie z.B. Fipronil mit weiteren Wirkstoffen oder von Pyrethroiden wie z.B. Flumethrin oder Deltamethrin mit weiteren Wirkstoffen beschrieben worden.

Die US 2002/0090387 und die US 5,567,429 beispielsweise betreffen die Kombination von Fipronil mit Insekten-Wachstums-Regulatoren (Insect-Growth-Regulator; IGRs) zur optimierten Bekämpfung von Flöhen bei Hunden und Katzen. Als IGRs werden z. B. Methoprene, Pyriproxyfen, Hydropren, Lufeneron, Triflumuron und Fenoxycarb genannt. Durch die Kombination dieser Wirkstoffklassen werden über zwei verschiedene Mechanismen (GABA-Hemmung und Wachstumshemmung der Larven) zum einen adulte Parasiten getötet und zum anderen Eier und Larven am weiteren Wachstum gehindert.

In der US 2003/0050327 wird die Kombination von Phenylpyrazolen wie unter anderem Fipronil mit Macrolid-Derivaten (macrocyclische Lactone) wie Ivermectin, Avermectin und Moxidectin in Spot-On Applikationen beschrieben.

In der DE 4 414 333 wird die prinzipielle Möglichkeit der Kombination von Wirkstoffen zur Bekämpfung von Parasiten, auch die Kombination von Pyridylpyrazolen mit z. B. Macroliden, IGRs, Phosphorsäureestern oder auch Pyrethroiden, erörtert. Die Gruppe der Pyridylpyrazole umfasst jedoch nicht das erfindungsgemäß besonders bevorzugte Phenylpyrazol-Derivat Fipronil. Die in dieser Druckschrift genannten Pyrethroide umfassen beispielsweise Deltamethrin, wohingegen Flumethrin nicht explizit genannt wird.

Die vorstehend bereits genannte US 5,232,940 umfasst in umfangreichen Listen wirksamer Phenylpyrazole unter anderen auch Fipronil (Verbindung Nr. 52). Die hierin genannten Phenylpyrazole können prinzipiell auch in zahlreichen möglichen Kombinationen mit diversen weiteren gegen Parasiten wirksamen Verbindungen kombiniert werden, wobei die Liste derartiger weiterer möglicher Wirkstoffe auch Wirkstoffe aus der Gruppe der Pyrethroide, insbesondere auch Deltamethrin, umfasst. Flumethrin wird nicht explizit genannt.

Auch in der US 6,472,417 werden Kombinationspräparate von Fipronil mit Pyrethroiden wie unter anderen Deltamethrin offenbart, wobei hierin ausschließlich die Verwendung als Insektizid gegen Termiten beschrieben wird.

Die WO 2001/35739 sowie die zugehörige Prioritätsanmeldung DE 19954394 betreffen Spot-on Präparate insbesondere auf Basis von Pyrethroiden, insbesondere Flumethrin, wobei prinzipiell auch Deltamethrin genannt wird. Dabei betrifft die Patentfamilie insbesondere die Bereitstellung geeigneter Wirkstoff-Formulierungen für die in Wasser schwer löslichen Pyrethroid-Wirkstoffe. Die Pyrethroide können mit diversen weiteren Wirkstoffen kombiniert werden, worunter prinzipiell auch N-Phenylpyrazole, zum Beispiel auch Fipronil, als mögliche Kombinationswirkstoffe genannt werden. Die Ausführungsbeispiele betreffen ausschließlich Kombinationspräparate auf Basis von Flumethrin in Kombination mit Nicotinyl-Insektiziden. Eine konkrete Kombination von Pyrethroiden wie Flumethrin oder Deltamethrin mit einem Wirkstoff aus der Gruppe der N-Phenylpyrazole wird in den Ausführungsbeispielen nicht offenbart.

Weitere Dokumente wie die US 2002/0177597, EP 2039248, WO 1998/23158, WO 2004/064522, WO 2009/071212 oder WO 2010/026370 betreffen Kombinationspräparate auf Basis ausgewählter spezifischer Wirkstoffe oder Verbindungsklassen in Kombination mit bekannten Wirkstoffen umfassend die Gruppen der Pyrethroide (z.B. Deltamethrin, Flumethrin) und der Pyrazole (z.B. Fipronil). Spezifische Kombinationen von Pyrethroiden und Pyrazolen werden darin nicht offenbart.

Präparate auf Basis einer Kombination von Pyrazolen und Pyrethroiden zur Bekämpfung von Ektoparasiten wie Zecken und Flöhen sind beispielsweise Gegenstand der WO 2008/080542 bzw. der DE 102006061538, worin die Liste der Pyrazole unter anderem auch Fipronil und die Liste der Pyrethroide unter anderem auch Deltamethrin und Flumethrin umfasst. Konkrete Ausführungsbeispiele, worin als Pyrazol Fipronil ausgewählt ist, werden nicht offenbart. Auch diese Druckschriften betreffen hauptsächlich die Bereitstellung geeigneter Wirkstoffformulierungen für die schwerlöslichen Pyrethroid-Wirkstoffe.

Desweiteren betrifft die EP 1624756 Kombinationspräparate auf Basis von Pyrethrinen oder Pyrethroiden in Kombination mit dem Synergisten MGK 264 (7-Methono-1H-isoindol-1,3(2H)-dion), wobei als bevorzugter Pyrethroid-Wirkstoff Flumethrin genannt wird. Derartige Kombinationspräparate können prinzipiell weitere Wirkstoffe wie zum Beispiel Pyrazole enthalten, wobei in der Liste der Pyrazole beispielsweise auch Fipronil genannt wird. Konkrete Ausführungsbeispiele betreffen lediglich Kombinationen von Flumethrin mit Imidacloprid bzw. Thiamethoxam oder Thiacloprid, also mit Wirkstoffen aus der Gruppe der Neonicotinoide, nicht jedoch mit solchen aus der Gruppe der Pyrazole.

Zur Anwendung zum Teil schwer wasserlöslicher Wirkstoffe, insbesondere solcher aus der Gruppe der Pyrethroide und Pyrethrine, in Form von dermal applizierbaren Flüssigformulierungen ist es notwendig, homogene Lösungen oder Emulsionen auf Basis von organischen Losungsmitteln und insektiziden Wirkstoffen herzustellen. Dazu werden die Wirkstoffe zumeist in organischen Losungsmitteln wie Isopropanol, 2-Butoxyethylacetat, Ethylenglykoldiacetat gelöst und gegebenenfalls mit weiteren Zusatzstoffen vermischt. Die Herstellung solcher Formulierungen ist in US 4,874,753, EP 137627 und GB 2135886 beschrieben. Die Nachteile der besagten Systeme liegen z.B. bei Verwendung von Wirkstoffen aus der Klasse der Pyrethrine sowie Pyrethroide, insbesondere α-Cyanopyrethroide darin, dass sie zu schweren Hautirritationen führen und ferner eine geringe Langzeitwirkung aufweisen.

Um den besagten Nachteil beispielsweise der bekannten Pyrethroide und Pyrethrine zu beheben, wird in AU 627847 und EP 413610 vorgeschlagen, diese Wirkstoffe in hochsiedenden Losungsmitteln wie Monopropylenglykol zu lösen, die zusätzlich noch natürliche, hautvertragliche Öle wie Pinienöl, Sonnenblumenöl oder Sojaöl enthalten.

Der WO 1991/13545 kann entnommen werden, dass gutwirksame, hautvertragliche Flüssigformulierungen hergestellt werden können, indem man die besagten Wirkstoffe in Mengen von >50% in aliphatischen Lösungsmitteln wie 2-(2-Butoxyethoxy)ethanol oder 2-(2-Methoxyethoxy)ethanol löst. Der Nachteil dieser Formulierungen liegt darin, dass sie den Einsatz größerer Wirkstoff-Mengen benötigen und zudem bei sensiblen Tierrassen zu Hautirritationen führen.

Um durch Einsatz geringer Wirkstoffmengen eine akzeptable biologische Wirkung zu erreichen, wird in der Patentschrift US 5,466,458 die Verwendung von Emulsionen auf Basis der besagten Wirkstoffe mit den langkettigen, aliphatischen Aminen oder Alkoholen wie Hexadecan-1-ol, 1-Octadecylamine vorgeschlagen. Die Verwendung der langkettigen Amine hat den Nachteil, dass sie die besagten Wirkstoffe im Laufe der Zeit abbauen. Die Formulierungen auf Basis langkettiger Alkohole weisen in den meisten Fällen keine ausreichende Langzeitwirkung auf.

Ferner wird in der WO 2001/35739 vorgeschlagen, die bezüglich Hautirritationen kritischen Pyrethroide, insbesondere α-Cyanopyrethroide, mit Polysiloxanen, die zusätzlich quarternäre Ammoniumgruppen enthalten, zu kombinieren. Diese Zubereitungsform hat jedoch den Nachteil, dass sie den Einsatz größerer Pyrethroid-Mengen erfordert.

Diese Tatsache kann in vielen Fällen zur Zieltier- oder Umwelt-Unverträglichkeit führen.

Der Literatur kann außerdem entnommen werden, dass man synthetische oder natürliche Pyrethroide mit organischen Synergisten wie Piperonylbutoxid (PBO), (2-(2-Ethylhexyl)-3a,4, 7,7a-tetrahydro-4,7-Methano-1 H-isoindol-1,3(2H)-dion (MGK 264), S,S,S-Tributylphosphorotrithioat (DEF) oder Synepirin kombinieren kann [so bespielsweise JOURNAL OF ECONOMIC ENTOMOLOGY, (1994 Aug) 87 (4) 879-84,1994; JOURNAL OF ECONOMIC ENTOMOLOGY, (1987 Aug) 35 80 (4) 728-32 oder India Chemosphere, (Nov., 1997) Vol. 35, No.10, pp. 2365-2374. ISSN: 0045-6535, Japanese Journal of Sanitary Zoology, (1995) Vol. 46, No.1, pp. 25-30. ISSN: 0424-7086. 1995 sowie J ECON ENTOMOL, (1987) 80 [6], 1117-1121. CODEN: JEENAI. ISSN: 0022-0493. 1987)]. Ferner kann der o.a. Literatur entnommen werden, dass die Wirksamkeit der Pyrethroid-haltigen Zubereitungen gegen adulte Flöhe verbessert werden kann, wenn man Pyrethroide mit den besagten Synergisten in Mengen 1:5 bis zu max. 1:20 kombiniert.

Außerdem ist beispielsweise bekannt aus DEP. ENTOMOL., UNIV. GEORGIA, COASTAL PLAIN EXP. STN., TEFLON, GA. 31793 oder India Chemosphere, (1998) 36/15 (3055-3060) 1998], dass eine maximale Wirksamkeitserhöhung bei einem Mengenverhältnis Wirkstoff zu Synergist von 1:5 erreicht wird (z.B. bei Permethrin/MGK-264 oder Fenvalerate/PBO).

Es ist weiterhin bekannt, dass Shampoos enthaltend Dipropylpyridin-2,5-dicarboxylat, MGK 264, Piperonyl-butoxid, und Pyrethrine zur Bekämpfung von Flöhen bei Kleintieren verwendet werden können [siehe beispielsweise 45 Wang I,-H,; Moorman R.; Burleson J.I-H. Wang, Journal of Liquid Chromatography and Related Technologies, (1996) 19/20 (3293-3304)].

Weiter ist aus der WO 2009/027506 A2 bekannt, in Fipronil-haltigen Spot-on Formulierungen zur Bekämpfung von Parasiten bei Tieren als Lösungsmittel Propylencarbonat bzw. Isopropylmyristat einzusetzen, wobei derartige Formulierungen außerdem auch Pyrethroide enthalten können.

Aus der WO 2008/030385 A2 ist darüber hinaus auch die konkrete Kombination dieser beiden Lösungsmittel bekannt.

Allerdings ergibt sich aus keinem dieser Dokumente die erfindungsgemäße vorteilhafte Auswahl der spezifischen Wirkstoffkombination mit Vitamin E Verbindungen (insbesondere in Gehalten ≥ 10 Gew.-%) zur Verbesserung der Verträglichkeit derartiger Präparate.

Vor dem Hintergrund dieses Stands der Technik ist es wünschenswert, die bekannten Formulierungen durch solche zu ersetzen, die eine hohe Hautverträglichkeit sowie toxikologische Unbedenklichkeit aufweisen, sich durch eine gute Langzeitwirkung von mehreren Wochen auszeichnen und eine gute Applikation auf dem Tier ermöglichen.

Es hat sich nun überraschend gezeigt, dass insektizide Wirkstoff-Formulierungen auf Basis von Phenylpyrazolen, insbesondere in Form von Kombinationspräparaten mit einem weiteren Insektizid aus der Gruppe der Pyrethroide, die Vitamin E oder ein Derivat davon wie insbesondere Vitamin E-Acetat, vorzugsweise in einer Menge ≥ 3,0 Gew.-% (bezogen auf die Gesamtzusammensetzung), bevorzugter ≥ 10 Gew.-% enthalten, deutlich verbesserte Effekte hinsichtlich Verträglichkeit und Langzeitwirkung gegenüber den bekannten Wirkstoff-Formulierungen verfügen.

Die Verwendung von Tocopherolen als Antioxidationsmittel in insektiziden Wirkstoff-Formulierungen wird beispielsweise bereits in den vorstehend genannten Druckschriften GB 2135886, WO 1991/13545 oder WO 2004/064522 erwähnt. Diese betreffen jedoch keine Wirkstoffkombinationen mit Wirkstoffen aus der Gruppe der Phenylpyrazole und der Pyrethroide.

In der WO 2004/064522 wird ein Anteil der Antioxidantien in der Gesamtzusammensetzung bis maximal 0,5 Gew.-% genannt. Höhere Tocopherolgehalte (≥ 3 Gew.-%) oder insbesondere auch die Verwendung von Tocopherolacetat werden nicht offenbart.

In der bereits genannten WO 2001/35739 sowie der EP 1624756 wird ebenfalls Tocopherol als ein mögliches Antioxidationsmittel genannt, wobei auch hier keine Kombinationspräparate von Phenylpyrazolen und Pyrethroiden, sondern nur solche auf Basis von Pyrethroiden (Flumethrin) mit Insektiziden aus anderen Wirkstoffklassen Gegenstand der Erfindung sind. Allerdings wird in beiden Druckschriften die prinzipielle Möglichkeit der Zugabe weiterer Kombinationswirkstoffe wie u.a. solcher aus der Gruppe der Phenylpyrazole (z.B. Fipronil) erwähnt. Es wird lediglich in der WO 2001/35739 ein konkretes Ausführungsbeispiel mit Tocopherol als Antioxidationsmittel aufgeführt, worin der Tocopherolanteil jedoch lediglich ca. 0,1 Gew.-% beträgt und worin ausschließlich Wirkstoffe aus der Gruppe der Pyrethroide (Flumethrin) und der Nicotinyl-Insektizide (Thiamethoxam) umfasst sind. Außerdem nennen beide Dokumente prinzipiell nur einen möglichen Anteil der Antioxidationsmittel bis maximal 2,5 Gew.-%. Eine explizite Kombination von Wirkstoffen aus der Gruppe der Phenyl-Pyrazole und der Pyrethroide mit ≥ 3 Gew.-% Vitamin E bzw. insbesondere mit Vitamin E Acetat ergibt sich somit auch aus diesen Dokumenten nicht.

Auch die WO 2010/026370 nennt prinzipiell Tocopherol als ein mögliches Antioxidationsmittel in insektiziden Wirkstoff-Formulierungen, wobei auch hierin der Anteil an Antioxidantien einen Gehalt von 1 Gew.-% nicht übersteigt. Dabei betrifft dieses Dokument außerdem Kombinationspräparate auf Basis eines Insektenwachtsumsregulators und/oder eines Pyrazol-Insektizids (z.B. Fipronil) und/oder eines Chloronicotinyl-Insektizids. Eine konkrete Auswahl des Pyrazol-Insektizids wie Fipronil in Kombination mit einem Pyrethroid-Wirkstoff und Tocopherol oder Tocopherolacetat kann damit auch diesem Dokument nicht explizit entnommen werden.

Hingegen ist aus der US 2011/071193 A1 sowie der zugehörigen Nachanmeldung WO 2011/038024 A1 ein Kombinationspräparat zur Schädlingsbekämpfung bei Tieren umfassend ein Phenylpyrazol (z.B. Fipronil) und ein Pyrethroid (z.B. Cyphenothrin), welche außerdem Antioxidationsmittel aus der Gruppe der Vitamin E Verbindungen enthalten können. Deltamethrin als mögliches Pyrethroid wird darin jedoch nicht genannt. Auch wird eine spezifische Kombination von Fipronil, Deltamethrin und Vitamin E Acetat nicht offenbart. Desweiteren wird hierin der Zusatz von Vitamin E Derivate vor dem Hintergrund deren antioxidativer Wirkung erwähnt. Es wird zwar angedeutet, dass Antioxidantien allgemein auch eine Verbesserung der Verträglichkeit bewirken können, dies wird jedoch weder auf eine konkrete Verbindungsgruppe der aufgelisteten Antioxidantien bezogen, noch auf eine spezifische Wirkstoffklasse spezifiziert. Insofern findet sich insbesondere kein konkreter Hinweis auf eine Verbesserung der Verträglichkeit speziell durch Vitamin E Derivate (insbesondere Vitamin E Acetat) für die eingesetzten Pyrethroide (insbesondere Deltamethrin, welches hierin gar nicht erwähnt wird). In diesen Dokumenten wird aus der Gruppe der Vitamin E Verbindungen Tocopherolnicotinat als bevorzugtes Antioxidationsmittel genannt, ohne hierfür einen Hinweis auf eine Verbesserung der Verträglichkeit zu geben.

Auch wird in diesen Dokumenten ein Zusatz der Antioxidantien bis lediglich 10 Gew.-% (üblicherweise weniger als 10 Gew.-%) offenbart. Hinweise für die Verwendung höherer Gehalte finden sich hierin nicht. Die Erfinder der vorliegenden Erfindung fanden jedoch überraschend, dass sich die vorteilhaften Effekte zur Verbesserung der Verträglichkeit insbesondere mit Gehalten von Vitamin E bzw. Vitamin E Acetat ab 10 Gew.-% oder höher zeigten.

Daneben finden sich in der Literatur Hinweise auf positive Effekte von Vitamin E und Derivaten davon bei Unverträglichkeiten auf insektizide Pyrethroid-Wirkstoffe. So beschreiben beispielsweise Flannigan et al. in BRITISH JOURNAL OF INDUSTRIAL MEDICINE (1985; 42:363-372) dass Vitamin E Acetat (dl-alpha-Tocopherolacetat) bei Parästhesien, die insbesondere durch Cyano-Pyrethroid-Wirkstoffe wie Fenvalerat ausgelöst wurden, positive Effekte zeigt. Die dargestellten Untersuchungen betreffen jedoch ausschließlich Unverträglichkeitsreaktionen bei Menschen, nicht jedoch bei Tieren wie Hunden oder Katzen. Darüber hinaus wird hierin lediglich ein Effekt des Vitamin E Acetats bei Auftreten von Parästhesien beschrieben. Ein Hinweis auf entsprechende positive Effekte bei gleichzeitiger Applikation anderer Pyrethroid-Wirkstoffe und Vitamin E Acetat in einem Kombinationspräparat im Sinne einer prophylaktischen Behandlung kann daraus nicht entnommen oder abgeleitet werden, Insbesondere werden entsprechende Effekte auch bei den erfindungsgemäß besonders bevorzugten Pyrethroiden Flumethrin und Deltamethrin nicht erwähnt. Bradberry et al. erwähnen in TOXICOL REV (2005; 24(2): 93-106) lediglich allgemein einen positiven Effekt einer topischen Applikation von dl-alpha Tocopherol Acetat bei Parästhesien nach Pyrethroid-induzierten Schädigungen beim Menschen.

Aldana et al. beschreiben in TOXICOL LETT. (2001 ; 125(1-3): 107-116) außerdem, dass oral verabreichtes alpha-Tocopherol Acetat positive Effekte bei hepatotoxischen Schädigungen nach i.p. Applikation von Cypermethrin (Pyrethroid-Insektizid) zeigt.

Malley et al. beschreiben außerdem in TOXICOL LETT. (1985; 29(1): 51-58), dass die dermale Vorbehandlung mit Vitamin E zu einer Reduzierung von Fenvalerat-induzierten Haut-Sensitivierungen bei Meerschweinchen führte. Eine vergleichbare Wirkung bei Nebenwirkungen, die durch Flumethrin oder Deltamethrin verursacht werden oder ein entsprechender Effekt bei der Behandlung von Hunden und Katzen wird nicht erwähnt, Auch ergibt sich kein Hinweis auf eine vorteilhafte Wirkung bei der gleichzeitigen Anwendung von im Sinne eines Kombinationspräparates.

### AUFGABENSTELLUNG

Die Aufgabe der vorliegenden Erfindung bestand somit darin, ein neues Mittel zur Bekämpfung von Parasiten an Tieren bereitzustellen, das über eine hohe Wirksamkeit, insbesondere eine hohe Langzeitwirkung, bei gleichzeitiger hoher Verträglichkeit, insbesondere Hautverträglichkeit, verfügt. Darüber hinaus bestand die Aufgabe darin, diese neuen insektiziden Mittel in geeigneten und verbesserten Wirkstoff-Formulierungen bereitzustellen, die über eine hohe Verträglichkeit, gute Applizierbarkeit und verbesserte Langzeitwirkung, insbesondere bei Verwendung als Spot-on Formulierung, verfügen.

### BESCHREIBUNG DER ERFINDUNG

Die Erfinder der vorliegenden Erfindung fanden überraschend, dass die vorliegende Aufgabe gelöst werden konnte durch Bereitstellung von Mitteln gemäß den Patentansprüchen 1 bis 13, die Fipronil als mindestens einen Wirkstoff aus der Gruppe der Phenylpyrazole in Kombination mit Vitamin E oder Derivaten davon wie insbesondere Vitamin E Acetat, vorzugsweise in einer Menge ≥ 3,0 Gew.-%, bevorzugter ≥ 10 Gew.-%, noch bevorzugter > 10 Gew.-% (bezogen auf die Gesamtzusammensetzung), sowie außerdem Deltamethrin als einen weiteren Wirkstoff aus der Gruppe der Pyrethroide sowie gegebenenfalls weitere zusätzliche Wirk-und/oder Hilfsstoffe enthalten.

Derartige verbesserte Mittel und deren vorteilhafte Effekte wurden im Stand der Technik bisher weder explizit beschrieben noch nahegelegt.

Diese verbesserten Effekte zeigten sich insbesondere auch bei der im Rahmen der vorliegenden Erfindung beanspruchten Ausführungsform, die Mittel zur Bekämpfung von Parasiten an Tieren betrifft, die zusätzlich zu Fipronil als mindestens einem Wirkstoff aus der Gruppe der Phenylpyrazole in Kombination mit Vitamin E oder einem Derivat davon außerdem Deltamethrin als mindestens einen Wirkstoff aus der Gruppe der Pyrethroide, sowie gegebenenfalls weitere zusätzliche Wirk- und/oder Hilfsstoffe enthalten.

Wirkstoffe aus der Gruppe der Phenylpyrazole (auch N-Phenylpyrazole oder N-Aryl-Pyrazole) umfassen prinzipiell solche wie aus US 2006/014802, WO 2005/090313, FR 2834288, WO 2009/828277, US 6,069, 157, WO 2000/31043, DE 9824487, WO 2009/804530, WO 2009/962903, EP 00933363, EP 0091 1329, WO 200/9856767, US 5,814,652, WO 2009/845274, WO 1998/40359, WO 2009/828279, WO 2009/828278, DE 9650197, WO 2009/824767, EP 00846686, EP 00839809, WO 2009/728126, EP 00780378, GB 02308365, US 5,629,335, WO 2009/639389, US 5,556,873, EP 00659745, US 5,321,040, EP 00511845, EP 234119, EP 2951 1 7, WO 1998/24769, US 5,232,940, EP 295117, EP 352944 bekannt sind.

Im Rahmen der vorliegenden Erfindung wird aus der Gruppe der Phenylpyrazol-Derivate Fipronil (5-Amino-3-cyano-1-(2,6-dichloro-4-trifluormethyl-phenyl)-4-trifluormethylsulphinylpyrazol) verwendet. Wirkstoffe aus der Gruppe der Pyrethroide umfassen sowohl natürliche als auch synthetische Pyrethroide.

Natürliche Pyrethroide umfassen insbesondere Pyrethrine wie Pyrethrin I und Pyrethrin II sowie Extrakte davon, sowie Pyrethrum und Derivate davon.

Synthetische Pyrethroide umfassen insbesondere sogenannte Typ I-Pyrethroide (ohne alpha-cyano-Gruppe) und Typ II-Pyrethroide (alpha-Cyanopyrethroide mit alpha-cyano-Gruppe) sowie auch Nicht-Ester Pyrethroide (z.B. Etofenprox). Diese unterscheiden sich im wesentlichen hinsichtlich ihrer akuten Wirkungen. Typ I führt im Tierversuch zum "T-Syndrom", so benannt nach dem auftretenden Tremor. Beim "T-Syndrom" werden auch Ataxie, erhöhte Erregbarkeit und Reiz-Überempfindlichkeit beobachtet. Typ 1-Pyrethroide umfassen beispielsweise

Pyrethroide vom Typ II bewirken ein "CS-Syndrom", das nach den charakteristischen Symptomen Choreoathetose (unwillkürliche langsame Bewegungen) und Speichelfluss benannt ist. Daneben treten hier auch ein grobschlägiger Tremor und klonische Krämpfe auf.

Synthetische Pyrethroide umfassen insbesondere Alphamethrin, Allethrin, Barthrin, Bioresmethrin, Biopermethrin, Cismethrin, Cyclethrin, Cypermethrin, Cyhalothrin, Cyfluthrin, Cyphenothrin, Deltamethrin, Dimethrin, Fenpropanate, Fenvalerate, Flumethrin, Fluvalinate, Indothrin, Permethrin, Phenothrin, Phthalthrin, Resmethrin, Tetramethrin, Sumithrin, Tralomethrin und Tralocythrin.

Besonders bevorzugt sind Typ II Pyrethroide (alpha-Cyanopyrethroide), wobei besonders bevorzugt Deltamethrin und Flumethrin sind. Im Rahmen der vorliegenden Erfindung wird Deltamethrin verwendet.

In einer weiteren bevorzugten Ausführungsform enthalten die erfindungsgemäßen Mittel zusätzlich einen oder mehrere Wirkstoffe aus der Gruppe der Entwicklungshemmer.

Entwicklungshemmer bzw. Insektenwachstumsregulatoren regulieren die Entwicklung von Larven und Verhindern deren Weiterentwicklung und Ausbildung zum adulten Schädling und damit deren Fortpflanzung. Entwicklungshemmer können beispielsweise aus der Gruppe der Juvenilhormone stammen. Entwicklungshemmer und Insektenwachstumsregulatoren umfassen beispielsweise Juvenilhormone wie Azadirachtin Diofenolan, Fenoxycarb, Hydroprene, Kinoprene, Methoprene, Pyriproxyfen, Tetrahydroazadirachtin, 4-Chlor-2-(2-chlor-2-methylpropyl)-5-(6-iod-3-pyridylmethoxy)pyridizin-3(2H)-on; sowie Chitin-Synthese-Inhibitoren wie Chlorfluazuron, Cyromazine, Diflubenzuron, Fluazuron, Flucycloxuron, Flufenoxuron, Hexaflumuron, Lufenuron, Tebufenozide, Teflubenzuron, Triflumuron. Bevorzugte Entwicklungshemmer sind Pyriproxifen und Methopren.

Alle im Rahmen der Erfindung genannten Wirkstoffe können außerdem durch ihre international bekannten Bezeichnungen gemäß "The Pesticide Manual"; 10th edition, 1994, Ed. Clive Tomlin, Great Britain definiert werden.

Die erfindungsgemäß verwendeten Wirkstoffe können gegebenenfalls in Abhängigkeit von Art und Anordnung der Substituenten in verschiedenen stereoisomeren Formen vorliegen, insbesondere als Enantiomere und Racemate, wobei sowohl die reinen Stereoisomere als auch deren Mischungen erfindungsgemäß eingesetzt werden können.

Gegebenenfalls können die erfindungsgemäßen Wirkstoffe auch in Form ihrer Salze eingesetzt werden, wobei insbesondere pharmazeutisch verwendbare Säureadditionssalze und basische Salze in Frage kommen, wie beispielsweise Salze von Mineralsäuren oder organischen Säuren (z. B. Carbonsäuren oder Sulfonsäuren), wie insbesondere Salzsäure, Schwefelsäure, Essigsäure, Glykolsäure, Milchsäure, Bernsteinsäure, Zitronensäure, Weinsäure, Methansulfonsäure, 4-Toluolsulfonsäure, Galacturonsäure, Gluconsäure, Embonsäure, Glutaminsäure oder Asparaginsäure. Pharmazeutisch verwendbare basische Salze umfassen beispielsweise Alkalisalze wie Natrium- oder Kaliumsalze und Erdalkalisalze wie Magnesium- und Calciumsalze.

Die erfindungsgemäßen Wirkstoffe können auch in Form ihrer Solvate, insbesondere Hydrate, eingesetzt werden, wovon sowohl die Solvate (insbesondere Hydrate) der Wirkstoffe selbst als auch die ihrer Salze umfasst sind.

Weitere Mittel betreffen:
1. Mittel enthaltend mindestens Fipronil und Vitamin E oder ein Derivat davon aus der Gruppe der Glykoside, Ester, Salze und Komplexe des Vitamin E (insbesondere Vitamin E Acetat) und mindestens Deltamethrin sowie gegebenenfalls weitere Wirk- und/oder Hilfsstoffe.
2. Mittel nach 1, die außerdem mindestens einen Wirkstoff aus der Gruppe der Entwicklungshemmer (insbesondere Pyriproxifen oder Methopren) enthalten.
3. Mittel nach 1 und 2, worin der Gehalt des Vitamin E's oder dem Derivat davon mindestens 10 Gew.-% bezogen auf die Gesamtzusammensetzung beträgt.

Besonders bevorzugt ist dabei eine Ausführungsform, enthaltend mindestens Fipronil als ein Phenylpyrazol und ≥ 10 Gew.-% Vitamin E oder ein Derivat davon aus der Gruppe der Glykoside, Ester, Salze und Komplexe des Vitamin E (insbesondere Vitamin E Acetat) und mindestens Deltamethrin als ein Pyrethroid sowie gegebenenfalls weitere Wirk- und/oder Hilfsstoffe.

Erfindungsgemäße Mittel enthalten eine Kombination von Fipronil, Vitamin E oder ein Derivat davon aus der Gruppe der Glykoside, Ester, Salze und Komplexe des Vitamin E, und Deltamethrin.

Aufgrund der besonders vorteilhaften Effekte einer derartigen Wirkstoffkombination umfasst die vorliegende Erfindung somit auch allgemein Mittel zur Bekämpfung von Parasiten an Tieren, enthaltend eine Kombination der Wirkstoffe Fipronil , Vitamin E oder ein Derivat davon aus der Gruppe der Glykoside, Ester, Salze und Komplexe des Vitamin E, und Deltamethrin.

Wie bereits erwähnt, sind insbesondere Wirkstoffe aus der Gruppe der Pyrethroide, wie beispielsweise auch Flumethrin und Deltamethrin, bekannt für ihre Haut- und Schleimhaut-irritierende Wirkung und das Auftreten von Nebenwirkungen in Form von Hautunverträglichkeitsreaktionen, beispielsweise Pruritus (Juckreiz), Erythembildung (Rötung) oder Parästhesien, Die Erfinder der vorliegenden Erfindung haben nun überraschend gefunden, dass derartige Nebenwirkungen und Unverträglichkeitsreaktionen, die durch die Applikation bzw. den dermalen (topischen, äußerlichen) Kontakt mit einer derartigen Wirkstoffkombination verursacht werden können durch die gleichzeitige Verabreichung von Vitamin E, insbesondere von Vitamin E Acetat (Tocopherolacetat) deutlich verringert bzw. ganz unterbunden werden können. Dabei erstreckt sich die Wirkung sowohl auf lokale als auch systemische Unverträglichkeitsreaktionen.

Insbesondere eine unmittelbare (gleichzeitige, simultane) Verabreichung der insektiziden Wirkstoffe mit dem Vitamin E bzw. Vitamin E Acetat in einem Kombinationspräparat hat sich dabei als besonders vorteilhaft herausgestellt. Durch diese gleichzeitige kombinierte Applikation kann die Bildung von Unverträglichkeitsreaktionen von vorneherein unterbunden bzw. reduziert werden, im Sinne einer prophylaktischen Behandlung, was vorteilhaft gegenüber einer aus dem Stand der Technik beschriebenen Nachbehandlung im Sinne einer akuten Behandlung ist, da dabei die Unverträglichkeitsreaktionen und damit eine Schädigung der betroffenen Haut- bzw. Schleimhautareale bereits erfolgt ist.

Insbesondere können durch die erfindungsgemäße Kombination der Pyrazol-Derivate und Pyrethroide mit Vitamin E (Vitamin E Acetat) topische bzw. dermale Nebenwirkungen wie Pruritus (Juckreiz) und Erythembildung (Rötung) sowie Alopezie und auch eine verstärkte Salivation der Schleimhäute verringert werden. Ein derartiger Effekt, insbesondere der positive Effekt zur Verringerung von topischen Nebenwirkungen wie Pruritus und Rötung, Alopezie und Salivation, von Vitamin E bei Pyrethroid-Applikation war aus dem Stand der Technik bisher nicht bekannt oder nahegelegt.

Im Rahmen der vorliegenden Erfindung umfasst der Begriff Vitamin E die Gruppe aller bisher entdeckten Vitamin E Verbindungen. Dabei handelt es sich um fettlösliche Substanzen mit häufig antioxidativen Wirkungen. Vitamin E ist Bestandteil der Membranen tierischer Zellen, wird jedoch nur von photosynthetisch aktiven Organismen wie Pflanzen und Cyanobakterien gebildet. Vier der bisher bekannten Vitamin-E-Formen werden auch als Tocopherole bezeichnet. Weitere vier Formen von Vitamin E werden Tocotrienole oder T3 genannt. Außerdem gibt es noch Tocomonoeole (T1) und MDT (marine derived tocopherols). Die am besten erforschte Form von Vitamin E ist alpha-Tocopherol ((2R)-2,5,7,8-Tetramethyl-2-[(4R,8R)-4,8,1 2-trimethyltridecyl]-3,4-dihydro-2H-chromen-6-ol):

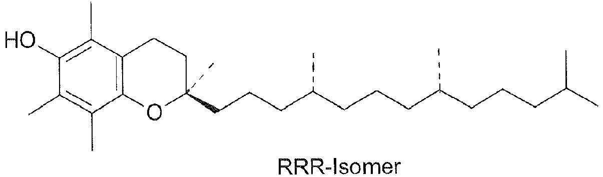

Weitere Formen sind beta-, gamma- und delta-Tocopherole. Auch die Tocotrienole, Tocomonoenole und MDT's können in alpha-, beta-, gamma- oder delta-Form vorliegen. Erfindungsgemäß umfasst sind außerdem alle Stereoisomeren sowie Mischungen davon.

Vitamin E Derivate betreffen im Rahmen der vorliegenden Erfindung insbesondere insbesondere Glykoside, Ester, Salze und Komplexe des Vitamin E. Besonders bevorzugt sind Ester des Vitamin E wie insbesondere Vitamin E Acetat bzw. Tocopherolacetat oder Tocopherylacetat (insbesondere alpha- Tocopherolacetat):

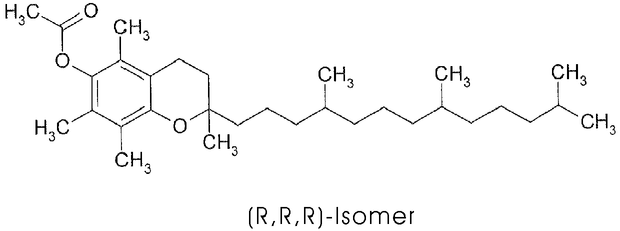

Bei alpha-Tocopherolacetat (Vitamin E Acetat) handelt es sich um ein synthetisches Vitamin E Derivat, das zur Gruppe der sogenannten Provitamine gehört. Aufgrund der drei Stereozentren des alpha-Tocopherolacetats ergeben sich acht stereoisomere alpha-Tocopherolacetate. Erfindungsgemäß umfasst sind alle acht Stereoisomeren sowie Mischungen wie dl-alpha Tocopherolacetat davon. Auch von Tocopherolacetat exisiteren außerdem die beta-, gamma- und delta-Formen mit jeweils acht Stereoisomeren.

Im Rahmen der vorliegenden Erfindung ist Vitamin E Acetat bzw. Tocopherolacetat (insbesondere alpha-Tocopherolacetat) besonders bevorzugt.

Im Rahmen der vorliegenden Erfindung sind insbesondere solche Kombinationspräparate bevorzugt, worin die Verwendung von Tocopherolnicotinat aus der Gruppe der Vitamin E Derivate ausgeschlossen ist.

Erfindungsgemäß haben sich vergleichsweise hohe Anteile an Vitamin E oder Derivaten davon, wie insbesondere Vitamin E Acetat, in den erfindungsgemäßen Mitteln als besonders wirksam erwiesen. Antioxidationsmittel werden üblicherweise in deutlich geringeren Mengen bis ca. 2,5 Gew.-% oder bis maximal 10 Gew.-% in insektiziden Wirkstoff-Formulierungen eingesetzt, womit auch bei Verwendung von beispielsweise Vitamin E in Funktion als Antioxidationsmittel dieses üblicherweise in Anteilen deutlich unter 1 Gew.-% eingesetzt wird.

Erfindungsgemäß wird Vitamin E oder seine Derivate vorzugsweise in einer Menge ≥ 3 Gew.-%, bevorzugter ≥ 5 Gew.-%, noch bevorzugter ≥ 7 Gew.-%, am bevorzugtesten ≥ 10 Gew.-%, jeweils bezogen auf die Gesamtzusammensetzung, in den erfindungsgemäßen Mitteln, beispielsweise in Mitteln gemäß den vorstehenden Ausführungsformen 1., 2. und 3., eingesetzt.

Ganz besonders bevorzugt ist es, einen Gehalt an Vitamin E oder seiner Derivate von > 10 Gew.-% einzusetzen, da hierdurch die erfindungsgemäß gewünschte Verbesserung der Verträglichkeit besonders gut erreicht wird. Dazu ist weiter bevorzugt, einen Vitamin E Gehalt von ≥ 1 2 Gew.-%, bevorzugter ≥ 15 Gew.-%, bevorzugter ≥ 18 Gew.-%, noch bevorzugter ≥ 20 Gew.-% einzusetzen.

Im Rahmen der vorliegenden Erfindung wurde außerdem überraschend gefunden, dass eine weitere Verbesserung der Verträglichkeit und Wirksamkeit der erfindungsgemäßen Wirkstoff-Kombination insbesondere durch Verwendung einer spezifischen Wirkstoff-Formulierung auf Basis einer Kombination eines aliphatischen cyclischen Carbonats und eines Spreitmittels erzielt werden kann.

Aliphatische cyclische Carbonate umfassen insbesondere Ehtylencarbonat, Propylencarbonat sowie Mischungen davon, wobei Propylencarbonat bevorzugt ist.

Spreitmittel umfassen beispielsweise oberflächenaktive Mittel wie Tenside, wie anionische Tenside (z.B. Natriumlaurylsulfat, Fettalkoholethersulfate und Monoethanolaminsalze von Mono-/Di-Alkylpolyglycoletherorthophosphorsäureestern), kationische Tenside (z.B. Cetyltrimethylammoniumchlorid), amphotere Tenside (z.B. Di-Natrium-N-Laurylaminodipropionat oder Lecithin), und nicht-ionische Tenside (z.B. polyoxyethyliertes Rizinusöl, polyoxyethyliertes Sorbitanmonooleat, Sorbitanmonostearat, Ethylalkohol, Glycerolmonosterat, Polyoxyethylenstearat und Alkylphenolpolyglycolether), sowie insbesondere polymere Tenside, beispielsweise solche auf Basis von Polymethoxysiloxanen, Silikone, Fette und Öle wie beispielsweise Silikonöle verschiedener Viskositäten; Fettsäureester wie Ethylstearat, Di-n-Butylester, Laurinsäurehexylester, Dipropylenglykolpelargonat, Ester einer verzweigten Fettsäure mittlerer Kettenlänge und gesättigten C16-C18-Fettalkoholen, Isopropylmyristat, Isopropylpalmitat, Capryl / Caprinsäure Ester aus gesättigten Fettalkoholen mit einer Kettenlänge von C12-C18, Isopropylstearat, Ölsäureoleylester, Ölsäuredecylester, Ethyloleat, Milchsäureethylester, wachsartige Fettsäureester, Dibutylphthalat, Adipinsäurediisopropylester und Ester-Gemische; Triglyceride auf Basis von Ölsäure, Palmitinsäure, Linolsäure, Stearinsäure, Caprylsäure und Caprinsäure wie insbesondere Capryl-/Caprinsäure-Triglycerid, TriglyceridGemische mit pflanzlichen Fettsäuren mit einer Kettenlänge von C8-C12 oder anderen speziell ausgewählten natürlichen Fettsäuren, Partialglyceridgemische von gesättigten und ungesättigten Fettsäuren und Mono- und/oder Diglyceride der C8-/C10-Fettsäuren; Fettalkohole, wie Isotridecylalkohol, 2-Octyldodecanol, Cetylstearylalkohol und Oleylalkohol; Fettsäuren wie Ölsäure Palmitinsäure, Linolsäure, Stearinsäure, Caprylsäure und Caprinsäure, Laktone wie Butyrolacton; Phospholipide und Phosphatidylcholine etc..

Besonders bevorzugte Spreitmittel sind Fettsäureester, wobei besonders bevorzugt Isopropylmyristat ausgewählt wird.

Bevorzugt sind somit die erfindungsgemäßen Wirkstoff-Formulierungen enthaltend eine Kombination von aliphatischem cyclischem Carbonat (insbesondere Propylencarbonat) und mindestens ein Spreitmittel aus der Gruppe der Fettsäureester (insbesondere Isopropylmyristat).

Die spezifische Auswahl einer Kombination von Propylencarbonat und einem Fettsäureester wie Isopropylmyristat hat sich dabei überraschend als besonders geeignet herausgestellt. Durch das Propylencarbonat kann einerseits eine gute Löslichkeit der gewählten Wirkstoffkombination erzielt werden. Allerdings ist aufgrund der hohen Polarität des Propylencarbonats lediglich eine unzureichende Verteilung und Applizierbarkeit gegeben. Durch die spezifische Auswahl des Spreitmittels Isopropyl-Myristat konnte eine optimale Stabilisierung der Wirkstoff-Formulierung und damit Verbesserung der Verteilbarkeit erreicht werden, die gegenüber anderen bekannten Spreitmitteln wie insbesondere gegenüber Silikonen bzw. Polysiloxanen, nicht-ionischen Tensiden oder Phosphatidylcholin insofern vorteilhaft ist, als damit kein Auskristallisieren der Formulierung und dadurch eine lokale Überdosierung am direkten Applikationsort erfolgt. Eine gute Verteilbarkeit wirkt sich neben einer Verbesserung der Verträglichkeit außerdem vorteilhaft auf die Langzeitwirkung aus, wie in den nachfolgenden Beispielen gezeigt wurde.

Aus dem Stand der Technik ist die Verwendung von aliphatischen cyclischen Carbonaten wie Propylencarbonat sowie von Spreitmitteln wie beispielsweise auch Fettsäureestern wie Isopropylmyristat in insektiziden Wirkstoff-Formulierungen prinzipiell bekannt, beispielsweise aus der WO 2001/35739 und DE 19954394, worin Wirkstoff-Formulierungen auf Basis von Polysiloxanen beschrieben werden, die unter anderem auch Propylencarbonate enthalten können, jedoch keinen Hinweis auf Spreitmittel wie Fettsäureester, insbesondere Isopropylmyristat geben, oder ganz allgemein auch aus der EP 1624756. Eine spezifische Kombination von Propylencarbonat mit Isopropylmyristat oder gar dieser Kombination mit der spezifischen Wirkstoffkombination gemäß der vorliegenden Erfindung kann keinem dieser Dokumente entnommen werden.

Auch die DE 102006061538 bzw. WO 2008/080542 sowie die GB 2457734 nennt cyclische Carbonate als mögliche Formulierungsbestandteile und zwar in Kombination mit aliphatischen cyclischen oder acyclischen Polyethern, wobei die DE 102006061538 bzw. WO 2008/080542 als Spreitmittel solche aus der Gruppe der Polysiloxane umfasst, wohingegen die GB 2457734 weder Spreitmittel aus der Gruppe der Silikone/Polysiloxane noch aus der Gruppe der Fettsäureester erwähnt. Die Verwendung von Tocopherol wird jeweils überhaupt nicht erwähnt.

Die Verwendung von Spreitmitteln aus der Gruppe der Silikone oder Polysiloxane ist aufgrund der beschriebenen Instabilität der Formulierung bei Applikation auf dem Tier nachteilig.

Die Verwendung von aliphatischen cyclischen oder acyclischen Polyethern ist nachteilig, da diese vergleichsweise polare Verbindungen darstellen, die sich negativ auf das Spreitverhalten der Wirkstoff-Formulierung auswirken, was wiederum nachteilig für die Verträglichkeit der Formulierung ist. Entsprechend sind erfindungsgemäß solche Wirkstoff-Formulierungen bzw. Mittel besonders bevorzugt, worin die Verwendung von Spreitmitteln aus der Gruppe der Silikone oder Polysiloxane und/oder aliphatischer cyclischer oder acyclischer Polyether ausgenommen ist.

In den erfindungsgemäßen verbesserten Wirkstoff-Formulierungen können die Wirkstoffe in folgenden Mengen enthalten sein:
Phenylpyrazole in einer Menge von mindestens 1,0 Gew.-%, bevorzugter mindestens 3,0 Gew.-%, noch bevorzugter mindestens 5,0 Gew.-%, ganz besonders bevorzugt mindestens 7,0 Gew.-% bzw. in einer Menge bis 20,0 Gew.-%, bevorzugter bis 17,0 Gew.-%, noch bevorzugter bis 15,0 Gew.-%, ganz besonders bevorzugt bis 12,0 Gew.-%;
Pyrethroide in einer Menge von mindestens 0,05 Gew.-%, bevorzugter mindestens 0,1 Gew.-%, noch bevorzugter mindestens 0,2 Gew.-%, ganz besonders bevorzugt mindestens 0,3 Gew.-% bzw. in einer Menge bis 7,0 Gew.-%, bevorzugter bis 5,0 Gew.-%, noch bevorzugter bis 3,0 Gew.-%, ganz besonders bevorzugt bis 1,5 Gew.-% enthalten ist enthalten sein kann.
Insbesondere sind die folgenden Wirkstoffbereiche bevorzugt: Phenylpyrazole 1,0 bis 20,0 Gew.-%, bevorzugter 3,0 bis 17,0 Gew.-%, noch bevorzugter 5,0 bis 15,0 Gew.-%, ganz besonders bevorzugt 7,0 bis 12,0 Gew.-% und
Pyrethroide 0,05 bis 7,0 Gew.-%, bevorzugter 0,1 bis 5,0 Gew.-%, noch bevorzugter 0,2 bis 3,0 Gew.-%, ganz besonders bevorzugt 0,3 bis 1,5 Gew.-%.

Dabei ist es einerseits durch die vorliegende Erfindung insbesondere möglich, durch die Kombination mit dem Vitamin E (oder seinen Derivaten) bei Verwendung vergleichsweise hoher Wirkstoffmengen dennoch eine hohe Verträglichkeit zu erzielen.

Andererseits kann jedoch auch bei Verwendung vergleichsweise geringer Wirkstoffmengen und einer damit verbundenen besseren Verträglichkeit speziell bei Verwendung der erfindungsgemäß bevorzugten Wirkstoff-Formulierung durch deren gute Applikation und Verteilbarkeit auf dem zu behandelnden Tier dennoch eine ausreichende bzw. gute Wirkung (insbesondere Langzeitwirkung) erzielt werden.

Desweiteren können die erfindungsgemäßen Mittel übliche Hilfsstoffe wie insbesondere übliche Lösungsmittel, Lösungsvermittler, Synergisten für die erfindungsgemäßen Wirkstoffe, Antioxidationsmittel, Konservierungsstoffe, Stabilisatoren, pH-Einstellungsmittel, Füllstoffe, Kristallisationsinhibitoren, Farbstoffe etc. enthalten.

Mögliche Lösungsmittel schließen insbesondere ein aromatische Alkohole wie Benzylalkohol, cyclische Carbonate wie Propylen- und Ethylencarbonat, Pyrrolidone wie Pyrrolidon-2, N-Methylpyrrolidon, N-Oktyl-, N-Butyl-pyrrolidon, niedrigsiedende Alkohole wie Isopropanol, Ethanol, höhere Alkohole wie n-Octylalkohol, Lanolinalkohol und n-Butanol, cyclische und acyclische Ketone wie Aceton, Methylethylketon und Cyclohexanon, Glykole wie Ethylen- und Propylenglykol, aliphatische cyclische oder acyclische Ether wie Tetrahydrofurfurylalkohol, Diethylenglykolmonoethylether, Dipropylenglykolmonopropylether und Glycofurol, Benzylbenzoat etc. Bevorzugt sind aliphatische cyclische Carbonate wie insbesondere Propylencarbonat, Ethanol, Benzylalkohol und Benzylbenzoat. Ganz besonders bevorzugt ist Propylencarbonat.

Als Stabilisatoren und Antioxidantien seien genannt Sulfite oder Metabisulfite wie Kaliummetabisulfit; organische Säuren wie Citronensäure, Ascorbinsäure, Äpfelsäure; Phenole, Butylhydroxytoluol (BHT), Butylhydroxyanisol, Vitamin E (Tocopherole) etc.

Die erfindungsgemäßen Mittel sind umweltverträglich und aufgrund der sehr geringen Toxizität anwenderfreundlich.

Die erfindungsgemäßen Mittel eignen sich zur Bekämpfung von parasitierenden Insekten die in der Tierhaltung und Tierzucht bei Haus- und Nutztieren sowie Zoo-, Labor-, Versuchs- und Hobbytieren vorkommen. Sie sind insbesondere gegen parasitäre Schädlinge wirksam, die ausgewählt sind aus der Gruppe der Ektoparasiten wie Insekten und Milben (z.B. Läuse, Zecken, Fliegen, Milben, Flöhe, Sandmücken etc.), insbesondere umfassend beispielsweise:
aus der Ordnung der Anoplura z. B. Haematopinus spp., Linognathus spp., Solenopotes spp., Pediculus spp., Pthirus spp.;
aus der Ordnung der Mallophaga z. B. Trimenopon spp., Menopon spp., Eomenacanthus spp., Menacanthus spp., Trichodectes spp., Felicola spp., Damalinea spp., Bovicola spp;
aus der Ordnung der Diptera in der Unterordnung Brachycera z. B. Chrysops spp., Tabanus spp., Musca spp., Hydrotaea spp., Muscina spp., Haematobosca spp., Haematobia spp., Stomoxys spp., Fannia spp., Glossina spp., Lucilia spp., Calliphora spp., Auchmeromyia spp., Cordylobia spp., Cochliomyia spp., Chrysomyia spp., Sarcophaga spp., Wohlfartia spp., Gasterophilus spp., Oesteromyia spp., Oedemagena spp., Hypoderma spp., Oestrus spp., Rhinoestrus spp., Melophagus spp., Hippobosca spp.;
aus der Ordnung der Diptera in der Unterordnung Nematocera z. B. Culex spp., Aedes spp., Anopheles spp., Culicoides spp., Phlebotomus spp., Simulium spp.;
aus der Ordnung der Siphonaptera z. B. Ctenocephalides spp., Echidnophaga spp., Ceratophyllus spp., Pulex spp.;
aus der Ordnung der Metastigmata z. B. Hyalomma spp., Rhipicephalus spp., Boophilus spp., Amblyomma spp., Haemaphysalis spp., Dermacentor spp., Ixodes spp., Argas spp., Ornithodorus spp., Otobius spp.;
aus der Ordnung der Mesostigmata z. B. Dermanyssus spp., Ornithonyssus spp., Pneumonyssus spp.;
aus der Ordnung der Prostigmata z. B. Cheyletiella spp., Psorergates spp., Myobia spp., Demodex spp., Neotrombicula spp.;
aus der Ordnung der Astigmata z. B. Acarus spp., Myocoptes spp., Psoroptes spp., Chorioptes spp., Otodectes spp., Sarcoptes spp., Notoedres spp., Knemidocoptes spp., Neoknemidocoptes spp., Cytodites spp., Laminosioptes spp..

Erfindungsgemäß besonders bevorzugt ist die Bekämpfung von parasitären Insekten aus der Gruppe der Ektoparasiten wie insbesondere von Zecken, Flöhen und Sandmücken.

Entsprechend betrifft eine bevorzugte Ausführungsform Mittel gemäß der vorliegenden Erfindung zur Verwendung in der prophylaktischen oder akuten Behandlung gegen Ektoparasiten, insbesondere gegen Zecken, Flöhe und Sandmücken.

Im Rahmen der vorliegenden Erfindung umfasst der Begriff der Nutz- und Zuchttiere beispielsweise Rinder, Pferde, Schafe, Schweine, Ziegen, Kamele, Wasserbüffel, Esel, Kaninchen, Damwild, Rentiere, Pelztiere wie z. B. Nerze, Chinchilla, Waschbär, Vögel wie z. B. Hühner, Gänse, Puten, Enten etc..

Der Begriff der Hobby- und Haustiere sowie Labor- und Versuchstiere umfasst erfindungsgemäß beispielsweise Mäuse, Ratten, Meerschweinchen, Goldhamster, Hunde, Katzen und Frettchen.

Bevorzugt sind die erfindungsgemäßen Mittel zur Verwendung in der Behandlung von Hunden, Katzen und Frettchen vorgesehen.

Dabei kann die Anwendung sowohl prophylaktisch als auch therapeutisch bzw. zur akuten Behandlung erfolgen.

Entsprechend betrifft eine weitere bevorzugte Ausführungsform Mittel gemäß der vorliegenden Erfindung zur Verwendung in der prophylaktischen oder akuten Behandlung von Hunden, Katzen und Frettchen.

Die Anwendung am Tier erfolgt erfindungsgemäß direkt oder bevorzugt in Form geeigneter Zubereitungen wie insbesondere den erfindungsgemäßen Wirkstoff-Formulierungen.

Dabei ist für eine optimale Wirkung, insbesondere die Repellentwirkung der Pyrethroid-Wirkstoffe, ein möglichst optimaler und großflächiger Hautkontakt vorteilhaft.

Besonders bevorzugt ist die Verwendung der erfindungsgemäßen Mittel zur äußerlichen, topischen bzw. dermalen Verwendung.

Geeignete Zubereitungen hierfür sind Lösungen oder Konzentrate zur Verabreichung nach Verdünnung zum Gebrauch auf der Haut oder in Körperhöhlen, Aufgussformulierungen, Gele, Emulsionen und Suspensionen, halbfeste Zubereitungen wie Formulierungen bei denen der Wirkstoff in einer Salbengrundlage oder in einer Öl-in-Wasser oder Wasser-in-Öl Emulsionsgrundlage verarbeitet ist, feste Zubereitungen wie Pulver, Premixe oder Konzentrate, Granulate, Pellets, Aerosole und wirkstoffhaltige Formkörper, die beispielsweise durch Auflösen und ggf. Verdünnen zum Gebrauch auf der Haut etc. verwendet werden. Erfindungsgemäß erfolgt die Applikation bevorzugt durch Sprühen, Gießen, Tropfen oder durch Applikation über Halsbänder für Katzen oder Hunde oder Frettchen.

Erfindungsgemäß ist insbesondere die Verwendung als Spot-on oder Pour-on Formulierung sowie die Applikation über Wirkstoff-Halsbänder bevorzugt.

Erfindungsgemäß umfasst ist auch die Verwendung der vorstehend beschriebenen neuen Mittel zur Bekämpfung von Parasiten wie insbesondere Ektoparasiten, insbesondere von Zecken, Flöhen und Sandmücken, beispielsweise durch Aufbringen und Behandeln von Ausrüstungsgegenständen oder Equipment aus der Tierhaltung wie z.B. von Tierkörben, Polstern, Bürsten, Käfigen, Ställen etc.. Dabei kann auch hier die Verwendung zur prophylaktischen als auch akuten Behandlung erfolgen.

Zur Herstellung der erfindungsgemäßen Mittel werden die gewünschten Bestandteile nach bekannten Verfahren in entsprechenden Mengen miteinander vermischt, beispielsweise unter Verwendung konventioneller Rührkessel oder anderer geeigneter Geräte.

Die Erfindung wird durch die folgenden Beispiele näher veranschaulicht.

### BEISPIELE:

### 1. Herstellbeispiele (erfindungsgemäße Mittel)

Spot-on Formulierung mit Fipronil, Deltamethrin und Vitamin E Acetat Zusammensetzung:

| **Herstellbeispiel 1 (BF-006)** | | **Herstellbeispiel 2 (BF-006.1)** | |
|---|---|---|---|
| Fipronil | 10,0 % | Fipronil | 10,0 % |
| Deltamethrin | 1,0 % | Deltamethrin | 0,2 % |
| Vitamin E Acetat | 10,0 % | Vitamin E Acetat | 10,0 % |
| Propylencarbonat | 35,0 % | Propylencarbonat | 35,8 % |
| Isopropyl-Myristat | 15,0 % | Isopropyl-Myristat | 15,0 % |
| Ethanol | 9,0 % | Ethanol | 9,0 % |
| Benzylbenzoat | 12,5 % | Benzylbenzoat | 12,5 % |
| Benzylalkohol | 7,5 % | Benzylalkohol | 7,5 % |

| **Herstellbeispiel 3 (BF-006.21)** | |
|---|---|
| Fipronil | 10,0 % |
| Vitamin E Acetat | 10,0 % |
| Propylencarbonat | 36,0 % |
| Isopropyl-Myristat | 15,0 % |
| Ethanol | 9,0 % |
| Benzylbenzoat | 12,5 % |
| Benzylalkohol | 7,5 % |

| **Herstellbeispiel 4 (BF-006.4)** | | **Herstellbeispiel 5 (BF-006.5)** | |
|---|---|---|---|
| Fipronil | 10,0 % | Fipronil | 10,0 % |
| Deltamethrin | 0,4 % | Deltamethrin | 0,4 % |
| Vitamin E Acetat | 15,0 % | Vitamin E Acetat | 18,0 % |
| Propylencarbonat | 32,6 % | Propylencarbonat | 30,6 % |
| Isopropyl-Myristat | 13,0 % | Isopropyl-Myristat | 12,0 % |
| Ethanol | 9,0 % | Ethanol | 9,0 % |
| Benzylbenzoat | 12,5 % | Benzylbenzoat | 12,5 % |
| Benzylalkohol | 7,5 % | Benzylalkohol | 7,5 % |

Die Herstellung der Zusammensetzungen erfolgte jeweils durch Vermischen der einzelnen Bestandteile miteinander bis zum Erhalt einer klaren, einphasigen Flüssigkeit.

### 2. Vergleichsformulierungen

Analog wurden die folgenden Vergleichsformulierungen erhalten:

| | **Vergleich 1** | **Vergleich 2** | **Vergleich 3** | **Vergleich 4** | **Vergleich 5** | **Vergleich 6** |
|---|---|---|---|---|---|---|
| Fipronil | 10 % | 10 % | 10 % | 10 % | 10 % | 10 % |
| Deltamethrin | 1 % | 1 % | 1 % | 1 % | 1 % | 1 % |
| Vitamin E Acetat | - | - | - | - | - | - |
| BHT | - | 2 % | - | - | - | 2 % |
| Propylencarbonat | 89 % | 73 % | 10 % | 40 % | 40 % | 87 % |
| Isopropyl-Myristat | - | - | - | 15 % | 15 % | - |
| Cyclomethicon 5NF (Silikon/Polysiloxan) | - | 10 % | 26 % | - | - | - |
| Abil B8832 (nichtionisches Tensid / Silikon) | - | 4 % | - | - | - | - |
| Phosal 53 MCT (Phosphatidylcholin) | - | - | 34 % | - | - | - |
| Ethanol | - | - | 19 % | 19 % | 9 % | - |
| Benzylbenzoat | - | - | - | 15 % | 15 % | - |
| Benzylalkohol | - | - | - | - | 10 % | - |

### 3. Stabilität der Formulierung und Spreitverhalten (Applizierbarkeit)

### Vergleichsformulierung 2

0,1 ml/kg der Formulierung aus Vergleichsformulierung Nr. 2 werden auf einen Versuchshund zwischen den Schulterblättern appliziert. Nach ca. 2 Stunden zeigen sich an der Applikationsstelle Kristalle an den Haarspitzen. Die Formulierung transportiert die Wirkstoffe nicht über das gesamte Tier, die Wirkstoffe konzentrieren sich an der Applikationsstelle auf.

### Vergleichsformulierung 3

0,1 ml/kg der Formulierung aus Vergleichsformulierung Nr. 3 werden auf einen Versuchshund zwischen den Schulterblättern appliziert. Nach ca. 2 Stunden zeigen sich an der Applikationsstelle Kristalle an den Haarspitzen. Die Formulierung transportiert die Wirkstoffe nicht über das gesamte Tier, die Wirkstoffe konzentrieren sich an der Applikationsstelle auf.

### Herstellbeispiel 1

0,1 ml/kg der erfindungsgemäßen Formulierung BF-006 werden auf einen Versuchshund zwischen den Schulterblättern appliziert. Sofort nach Applikation verteilt sich die Lösung vollständig über das gesamte Tier. Kristalle sind zu keinem Zeitpunkt zu beobachten.

### Ergebnis:

Es zeigte sich, dass für die erfindungsgemäß gewählte Wirkstoffkombination die bekannten Spreitmittel aus der Gruppe der Silikone/Polysiloxane, Tenside sowie Phosphatidylcholin nicht geeignet sind, um eine Formulierung mit guter Applizierbarkeit und hoher Verteilbarkeit auf dem Tier zu ermöglichen. Für eine optimale Verträglichkeit und Langzeitwirkung der Wirkstoff-Formulierung ist eine optimale Verteilung insofern von Bedeutung, als eine Kristallisation und Aufkonzentration der Wirk- und Hilfsstoffe am Applikationsort, wie bei den Vergleichsformulierungen beobachtet, zu einer lokalen Überdosierung und damit einer schlechteren Verträglichkeit und geringeren Langzeitwirkung führt.

### 4. Wirksamkeitsnachweis

### Wirksamkeit gegen Flöhe und Zecken

Sechs Katzen wurden in zwei Gruppen randomisiert und am Tag -1 mit ca. 100 Flöhen und 50 lebenden erwachsenen Zecken infestiert. Am Tag 0 wurden drei Katzen (Nr. 149, 96 und 168) mit der Spot-On-Formulierung BF-006.1 nach Herstellbeispiel 2 (0,1 ml/kg Tiergewicht) behandelt, drei Tiere (Nr. 66, 53 und 47) dienten als unbehandelte Kontrolle.

Reinfestationen, das heißt, erneute Infestationen mit Flöhen und Zecken wurden an folgenden Tagen durchgeführt: Tag 5, Tag 12, Tag 19 und Tag 26.

Alle sechs Katzen wurden an folgenden Tagen gekämmt und hinsichtlich eines Befalls auf Flöhe und Zecken untersucht: Tag 2, Tag 7, Tag 14, Tag 21 und Tag 28.

Die Befallszahlen sowie die Wirksamkeiten sind in Tabelle 1 (Floh) und Tabelle 2 (Zecke) dargestellt:

**Tabelle 1: Wirksamkeit gegen Flöhe**

| | **Anzahl lebende Flöhe auf dem Tier** | | | | | |
|---|---|---|---|---|---|---|
| **Katze Nr.** | | **Tag 2** | **Tag 7** | **Tag 14** | **Tag 21** | **Tag 28** |
| | **149** | 1 | 0 | 0 | 0 | 2 |
| | **96** | 0 | 0 | 0 | 0 | 11 |
| | **168** | 0 | 0 | 0 | 0 | 0 |
| | **66** | 81 | 84 | 91 | 10 | 91 |
| | **53** | 67 | 73 | 87 | 79 | 93 |
| | **47** | 79 | 71 | 78 | 67 | 58 |
| **Wirksamkeit in %** | | **99,6** | **100** | **100** | **100** | **94,6** |

**Tabelle 2: Wirksamkeit gegen Zecken**

| | | **Anzahl lebender festgesogener Zecken auf dem Tier** | | | | |
|---|---|---|---|---|---|---|
| **Katze Nr.** | | **Tag 2** | **Tag 7** | **Tag 14** | **Tag 21** | **Tag 28** |
| | **149** | 0 | 0 | 0 | 0 | 0 |
| | **96** | 1 | 3 | 0 | 0 | 1 |
| | **168** | 0 | 0 | 0 | 0 | 0 |
| | **66** | 0 | 5 | 22 | 7 | 3 |
| | **53** | 0 | 2 | 13 | 13 | 24 |
| | **47** | 1 | 3 | 4 | 4 | 1 |
| **Wirksamkeit in %** | | **0** | **70** | **100** | **100** | **96,4** |

### 5. Untersuchung zur Verträglichkeit

Sechs Hunde und sechs Katzen wurden hinsichtlich der lokalen und systemischen Verträglichkeit der erfindungsgemäßen Wirkstoff-Kombination mit und ohne Vitamin E untersucht.

Jeweils drei Hunde und drei Katzen erhielten das Spot-on BF-006 (entsprechend Herstellbeispiel 1) mit Vitamin E (0,3 ml/kg Tiergewicht), jeweils drei Hunde und drei Katzen erhielten das entsprechende Spot on ohne Vitamin E.

Alle Tiere wurden danach nach 30', 1 h, 2h, 4h, 8h, 1 2h und 24h hinsichtlich der lokalen und systemischen Verträglichkeit der Spot-on Formulierungen beobachtet und untersucht.

Tabelle 3 zeigt die Verträglichkeiten in der Zusammenfassung der Hunde, Tabelle 4 die der Katzen:

**Tabelle 3: Verträglichkeit Hund**

| **Hunde Nr.** | **Spot on** | **Systemische Verträglichkeit** | **Lokale Verträglichkeit nach 24 h** |
|---|---|---|---|
| 42 | Ohne Vitamin E (VF005, 0,3ml/kg) | Juckreiz: + + nach 2h | Gerötet: ++; Alopezie ca. 50 Cent-Stück groß |
| 44 | | Juckreiz: + nach 4h | Gerötet: + |
| 45 | | Juckreiz: +++ nach 30'; starke Salivation | Gerötet: +; Alopezie ca. 50 Cent-Stück groß |
| 36 | Mit 10% Vitamin E (BF-006, 0,3 ml/kg) | gut | gut |
| 47 | | gut | gut |
| 51 | | gut | gut |

**Tabelle 4: Verträglichkeit Katze**

| **Katze Nr.** | **Spot on** | **Systemische Verträglichkeit** | **Lokale Verträglichkeit nach 24 h** |
|---|---|---|---|
| 22 | Ohne Vitamin E (VF005, 0,3ml/kg) | Juckreiz: + nach 30' | Gerötet: + + |
| 27 | | gut, leichte Salivation | Gerötet: + |
| 39 | | Juckreiz: + + nach 2h; starke Salivation | Gerötet: ++; Alopezie ca. 2€-Stück groß |
| 25 | Mit 10 % Vitamin E (BF-006, 0.3 ml/kg) | gut | gut |
| 29 | | gut, leichte Salivation | gut |
| 31 | | gut | gut |

| | | | |
|---|---|---|---|
| + leicht ++ mittel +++ stark | | | |

### 6. Vergleichsversuche zum Stand der Technik

Vergleich mit Zusammensetzungen nach dem Stand der Technik (US 2011/0071193)

### Erfindungsgemäße Formulierung BF-006.3 (#1)

| | w in (%) |
|---|---|
| Fipronil | 10,0 |
| Deltamethrin | 0,4 |
| Propylencarbonat | 35,6 |
| Ethanol | 9,0 |
| Isopropylmyristat | 15,0 |
| Benzylbenzoat | 12,5 |
| Benzylalkohol | 7,5 |
| Vitamin E acetat | 10,0 |

Dosierung 0,1 ml/kg Tiergewicht

### Formulierung nach dem Stand der Technik (analog US2011/0071193 A1) (#2)

| | w in (%) |
|---|---|
| Fipronil | 10,0 |
| Cyphentoin | 0,4 |
| Vitamin E nicotinat | 0,5 |
| Diethylenglylolmonoethylether | 89,1 |

Dosierung 0,1 ml/kg Tiergewicht

### Applikationsversuch der beiden Formulierungen #1 und #2 an Katzen

Dosierung 0,3ml/kg

| **Katze 1 / 1,35 ml** | | | | | | |
|---|---|---|---|---|---|---|
| | **Nach 24 h** | | **Nach 48 h** | | **Nach 72 h** | |
| **Formulierung** | **#1** | **#2** | **#1** | **#2** | **#1** | **#2** |
| Auftragsstelle sichtbar | Ja | Ja | Nein | Ja | Nein | Ja |
| Haare verklebt | Ja | Ja | Nein | Ja | Nein | Ja |
| Wirkstoffkristalle am Auftragungsort | Nein | Nein | Nein | Ja | Nein | Ja |
| | | | | | | |

| **Katze 2 / 1,50 ml** | | | | | | |
|---|---|---|---|---|---|---|
| Auftragsstelle sichtbar | Ja | Ja | Nein | Ja | Nein | Ja |
| Haare verklebt | Ja | Ja | Nein | Ja | Nein | Ja |
| Wirkstoffkristalle am Auftragungsort | Nein | Ja | Nein | Ja | Nein | Ja |
| | | | | | | |

| **Katze 3 / 1,80 ml** | | | | | | |
|---|---|---|---|---|---|---|
| Auftragsstelle sichtbar | Ja | Ja | Ja | Ja | Nein | Ja |
| Haare verklebt | Ja | Ja | Ja | Ja | Nein | Ja |
| Wirkstoffkristalle am Auftragungsort | Nein | Ja | Nein | Ja | Nein | Ja |

Anhand dieser Daten wird ersichtlich, dass die erfindungsgemäße Formulierung BF-006.3 **(#1)** im Vergleich zur Formulierung analog US 2011/00711193 A1 **(#2)** insbesondere die folgenden entscheidenden Vorteile aufweist:
1. Die wirkstoffhaltige Lösung verteilt sich deutlich besser über das Tier, so dass hieraus auch eine bessere Wirkstoffverteilung resultiert, was einen größeren Schutz des Tieres gewährleistet und auch zu einer besseren Verträglichkeit führt, da die Wirkstoffe über eine größere Fläche verteilt sind.
2. Die Formulierung analog US 201 1/0071 193 A1 **(#2)** hat starke Tendenz zur Auskristallisation. Am Applikationsort bilden sich spätestens nach 24 h Wirkstoffkristalle, die in den Haarspitzen zu finden sind. Durch die Auskristallisation ist die Verteilung der Wirkstoffe über das Tier nicht mehr vollständig, das bedeutet, dass die wirksame Dosis unterschritten wird. Außerdem besteht hierdurch eine höhere Kontaminationsgefahr für den Tierhalter.

## Patentansprüche

1. Mittel enthaltend
- Fipronil als einen Wirkstoff aus der Gruppe der Phenylpyrazole,
- Deltamethrin als einen Wirkstoff aus der Gruppe der Pyrethroide und
- Vitamin E oder ein Derivat davon aus der Gruppe der Glykoside, Ester, Salze und Komplexe des Vitamin E, sowie
- gegebenenfalls zusätzlich weitere Wirk- und/oder Hilfsstoffe, zur Verwendung in der Bekämpfung von Parasiten an Tieren.

2. Mittel zur Verwendung nach Anspruch 1, worin das Vitamin E oder das Derivat davon in einer Menge ≥ 10 Gew.-%, bezogen auf die Gesamtzusammensetzung, enthalten ist.

3. Mittel zur Verwendung nach einem der Ansprüche 1 bis 2, enthaltend zusätzlich einen oder mehrere Wirkstoffe aus der Gruppe der Entwicklungshemmer und/oder Hilfsstoffe aus der Gruppe der aliphatischen cyclischen Carbonate und der Spreitmittel.

4. Mittel zur Verwendung nach einem der vorhergehenden Ansprüche, enthaltend zusätzlich einen oder mehrere Wirkstoffe aus der Gruppe der Entwicklungshemmer, die ausgewählt sind aus der Gruppe bestehend aus Pyriproxifen und Methopren.

5. Mittel zur Verwendung nach einem der vorhergehenden Ansprüche, enthaltend mindestens einen Hilfsstoff aus der Gruppe der aliphatischen cyclischen Carbonate, ausgewählt aus Propylencarbonat.

6. Mittel zur Verwendung nach einem der vorhergehenden Ansprüche, enthaltend mindestens einen Hilfsstoff aus der Gruppe der Spreitmittel, ausgewählt aus der Gruppe der Fettsäureester.

7. Mittel wie in Anspruch 6 definiert, worin das Spreitmittel aus der Gruppe der Fettsäureester Isopropylmyristat ist.

8. Mittel wie in einem der vorhergehenden Ansprüche definiert zur Verwendung in der prophylaktischen oder akuten Behandlung gegen Ektoparasiten.

9. Mittel wie in Anspruch 8 definiert, worin die Ektoparasiten Zecken, Flöhe und Sandmücken sind.

10. Mittel wie in einem der vorhergehenden Ansprüche definiert zur Verwendung in der prophylaktischen oder akuten Behandlung von Hunden, Katzen und Frettchen.

11. Mittel zur Verwendung nach einem der vorhergehenden Ansprüche zur topischen Anwendung.

12. Mittel zur Verwendung nach einem der vorhergehenden Ansprüche zur Anwendung als Spot-on Formulierung.

13. Mittel wie in einem der vorhergehenden Ansprüche definiert, worin das Vitamin E oder das Derivat davon Vitamin E Acetat oder alpha-Tocopherolacetat ist.

## Claims

1. An agent, comprising
- fipronil as an active substance from the group of phenylpyrazoles,
- deltamethrin as an active substance from the group of pyrethroids, and
- vitamin E or a derivative thereof, which are selected from the group consisting of glycosides, esters, salts and complexes of vitamin E, as well as additionally
- optionally further active and/or auxiliary substances,
for the use in the control of parasites on animals.

2. The agent for the use according to claim 1, wherein the vitamin E or the derivative thereof is contained in a quantity ≥ 10 % by wt., based on the total composition.

3. The agent for the use according to any one of the claims 1 or 2, additionally comprising one or more active substances from the group of development inhibitors and/or auxiliary substances from the group of aliphatic cyclic carbonates and of spreading agents.

4. The agent for the use according to any one of the preceding claims, additionally comprising one or more active substances from the group of development inhibitors, which are selected from the group consisting of pyriproxifen and methoprene.

5. The agent for the use according to any one of the preceding claims, comprising at least one auxiliary substance from the group of aliphatic cyclic carbonates, which are selected from propylene carbonate.

6. The agent for the use according to any one of the preceding claims, comprising at least one auxiliary substance from the group of spreading agents, which are selected from the group of fatty acid esters.

7. The agent as defined in claim 6, wherein the spreading agent from the group of fatty acid ester is isopropyl myristate.

8. The agent as defined in any one of the preceding claims for the use in the prophylactic or acute treatment against ectoparasites.

9. The agent as defined in claim 8, wherein the ectoparasites are selected from the group consisting of ticks, fleas and sand flies.

10. The agent as defined in any one of the preceding claims for the use in the prophylactic or acute treatment of dogs, cats and ferrets

11. The agent for the use according to any one of the preceding claims for topical application.

12. The agent for the use according to any one of the preceding claims for application as a spot-on formulation.

13. The agent as defined in any one of the preceding claims, wherein the vitamin E or the derivative thereof is selected from amongst vitamin E acetate or alpha-tocopheryl acetate.

## Revendications

1. Produit contenant
- du fipronil en tant que principe actif du groupe des phénylpyrazoles,
- de la deltaméthrine en tant que principe actif du groupe des pyréthroïdes et
- de la vitamine E ou un dérivé de celle-ci du groupe des glycosides, des esters, des sels et des complexes de la vitamine E ainsi que
- le cas échéant, supplémentairement d'autres substances actives et/ou auxiliaires
pour l'utilisation dans la lutte antiparasitaire chez les animaux.

2. Produit pour l'utilisation selon la revendication 1, dans lequel la vitamine E ou le dérivé de celle-ci est contenu en une quantité de ≥ 10 % en poids par rapport au poids total de la composition.

3. Produit pour l'utilisation selon l'une des revendications 1 à 2, contenant supplémentairement un ou plusieurs principes actifs du groupe comprenant des inhibiteurs et/ou des substances auxiliaires du groupe comprenant des carbonates cycliques aliphatiques et des agents d'étalement.

4. Produit pour l'utilisation selon l'une des revendications précédentes, contenant supplémentairement un ou plusieurs principes actifs du groupe comprenant des inhibiteurs, qui sont sélectionnés dans le groupe consistant en des pyriproxyfènes et des méthoprènes.

5. Produit pour l'utilisation selon l'une des revendications précédentes, contenant au moins une substance auxiliaire du groupe de carbonates cycliques aliphatiques, sélectionnée du carbonate de propylène.

6. Produit pour l'utilisation selon l'une des revendications précédentes, contenant au moins une substance auxiliaire du groupe comprenant des agents d'étalement, sélectionnée du groupe d'esters d'acides gras.

7. Produit comme défini dans la revendication 6, dans lequel l'agent d'étalement du groupe d'esters d'acides gras est du myristate d'isopropyle.

8. Produit comme défini dans une des revendications précédentes pour l'utilisation dans le traitement prophylactique ou aigu contre les ectoparasites.

9. Produit comme défini dans la revendication 8, dans lequel des ectoparasites sont des tiques, des puces et des mouches des sables.

10. Produit comme défini dans une des revendications précédentes pour l'utilisation dans le traitement prophylactique ou aigu des chiens, des chats et des furets.

11. Produit pour l'utilisation selon l'une des revendications précédentes pour l'application topique.

12. Produit pour l'utilisation selon l'une des revendications précédentes pour l'application en tant que formulation Spot-on.

13. Produit comme défini dans une des revendications précédentes, dans lequel la vitamine E ou le dérivé de celle-ci est l'acétate de vitamine E ou l'acétate d'alpha-tocophérol.
